(19) Europäisches Patentamt
European Patent Office
Office européen des brevets

(11) Veröffentlichungsnummer: **0 008 652**
**B1**

(12) **EUROPÄISCHE PATENTSCHRIFT**

(45) Veröffentlichungstag der Patentschrift: **22.09.82**

(21) Anmeldenummer: **79102567.9**

(22) Anmeldetag: **20.07.79**

(51) Int. Cl.³: **C 07 D 401/04,**
C 07 D 239/48,
C 07 D 498/04
//A61K31/505, (C07D498/04, 271/00, 239/00)

(54) **Neue Zwischenprodukte und deren Verwendung zur Herstellung von neuen Oxadiazolopyrimidinderivaten.**

(30) Priorität: **21.07.78 CH 7910/78**

(43) Veröffentlichungstag der Anmeldung:
**19.03.80 Patentblatt 80/6**

(45) Bekanntmachung des Hinweises auf die Patenterteilung:
**22.09.82 Patentblatt 82/38**

(84) Benannte Vertragsstaaten:
**AT BE CH DE FR GB IT LU NL SE**

(56) Entgegenhaltungen:
**DE - A1 - 2 804 518**
**DE - A1 - 2 804 519**

(73) Patentinhaber: **F. HOFFMANN-LA ROCHE & CO. Aktiengesellschaft**
**CH-4002 Basel (CH)**

(72) Erfinder: **Muller, Jean-Claude, Dr.**
**27 rue Albert Schweitzer Parc d'Entremont**
**F-68170 Rixheim (FR)**
Erfinder: **Ramuz, Henri, Dr.**
**Rheinparkstrasse 3**
**CH-4127 Birsfelden (CH)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 652 B1

Neue Zwischenprodukte und deren Verwendung zur Herstellung von neuen Oxadiazolopyrimidinderivaten

Die vorliegende Erfindung betrifft neue Verbindungen der allgemeinen Formel

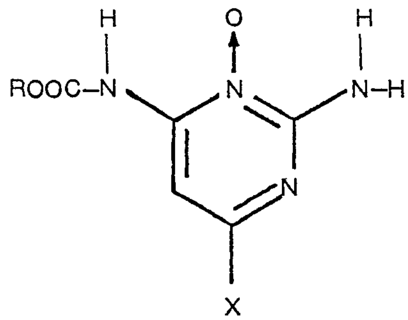

I

worin R $C_1$—$C_8$-Alkyl order $C_1$—$C_8$-Alkoxy-$C_1$—$C_8$-alkyl und X 1, 2, 5, 6-Tetrahydropyridin-1-yl oder Chlor bedeuten.

Die Verbindungen der obigen Formel I sind wertvolle Zwischenprodukte und können zur Herstellung von neuen Oxadiazolopyrimidinderivaten der allgemeinen Formel

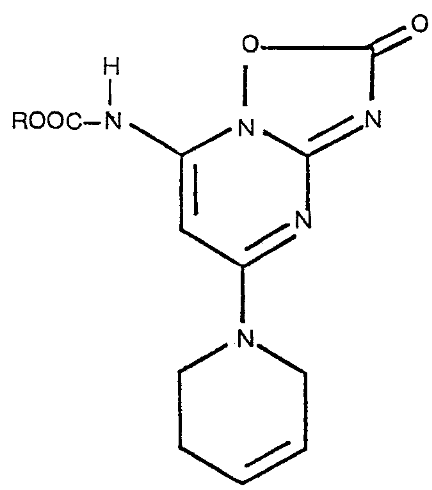

II

worin R die obige Bedeutung besitzt, sowie Salzen davon verwendet werden.

Der in dieser Beschreibung verwendete Ausdruck "$C_1$—$C_8$-Alkyl" — allein oder in Kombination — bezieht sich auf geradkettige und verzweigte, gesättigte Kohlenwasserstoffreste, wie z.B. Methyl, Aethyl, n-Propyl, Isopropyl, n-Butyl, Isobutyl, tert. Butyl und dgl. "$C_1$—$C_8$-Alkoxy" bezieht sich auf Alkyläthergruppen, in denen der Alkylrest die obige Bedeutung besitzt.

Von den Verbindungen der Formel I sind diejenigen bevorzugt, in denen R $C_1$—$C_8$-Alkyl bedeutet. Besonders bevorzugt sind diejenigen, in denen R Alkyl mit 1—4 Kohlenstoffatomen bedeutet. Ganz besonders bevorzugt sind diejenigen Verbindungen der Formel I, worin R Methyl bedeutet.

Die Verbindungen der Formel I, worin X 1, 2, 5, 6-Tetrahydropyridin-1-yl bedeutet, können so hergestellt werden, dass man 2,4-Diamino-6-[3,6-dihydro-1(2H)-pyridyl]pyrimidin-3-oxid bzw. die Tautomeren 6-Amino-4-[3,6-dihydro-1(2H)-pyridyl]-1,2-dihydro-1-hydroxy-2-imino-pyrimidin und 2-Amino-4-[3,6-dihydro-1(2H)-pyridyl]-1,6-dihydro-1-hydroxy-6-imino-pyrimidin der Formeln

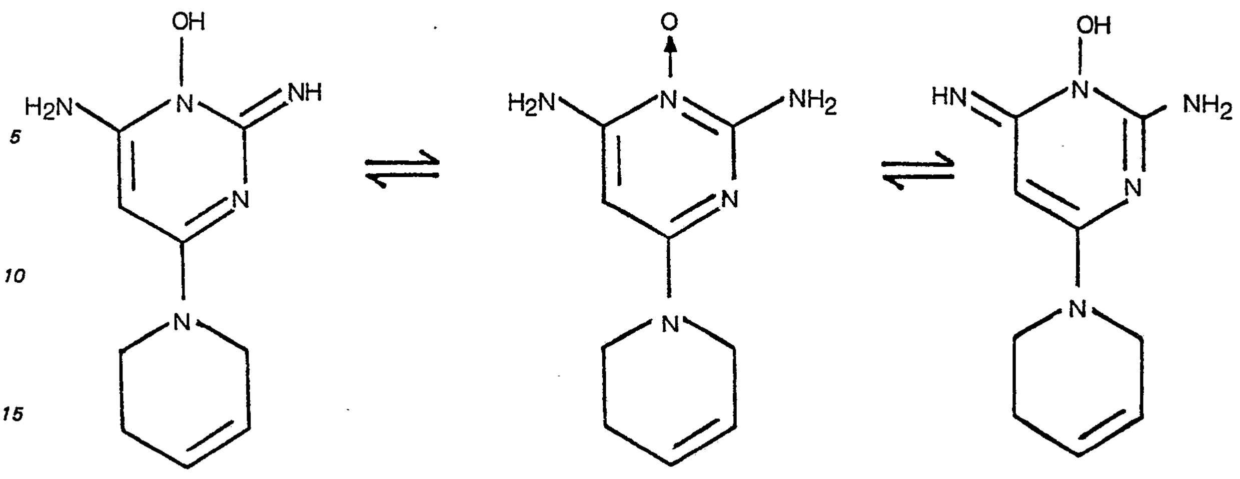

IV III V

mit einem Chlorameisensäureester der allgemeinen Formel

Cl—COOR VI

worin R die obige Bedeutung besitzt, umsetzt. Die Umsetzung erfolgt in einem inerten Lösungsmittel oder Lösungsmittelgemisch in Gegenwart eines säurebindenden Mittels. Für den vorliegenden Zweck geeignete Lösungsmittel sind chlorierte Kohlenwasserstoffe wie Methylen-chlorid oder Chloroform, Aether wie Diäthyläther, Tetrahydrofuran oder Dioxan, Dimethylformamid und dgl. oder Gemische davon. Die Umsetzung kann auch in einem wasserhaltigen Lösungsmittel bzw. in Gegenwart von Wasser in einem Zweiphasensystem wie z.B. Methylenchlorid/Wasser durchgeführt werden. Als säurebindende Mittel kommen Basen wie Triäthylamin, Aethyldiisopropylamin, Dimethylamin, Pyridin, Alkalihydroxyde und dgl. in Frage. Wird die Umsetzung in Gegenwart einer flüssigen Base durchgeführt, so kann diese auch als Lösungsmittel dienen. Die Umsetzung wird zweckmässig bei Temperaturen zwischen —10°C und Raumtemperatur durchgeführt, vorzugsweise zwischen 0° und 10°C.

Wird die Umsetzung mit 1 Moläquivalent Chlorameisensäureester durchgeführt, so erhält man direkt die erwünschte Verbindung der Formel I, worin X 1,2,5,6-Tetrahydropyridin-1-yl bedeutet, d.h. die Verbindung der allgemeinen Formel

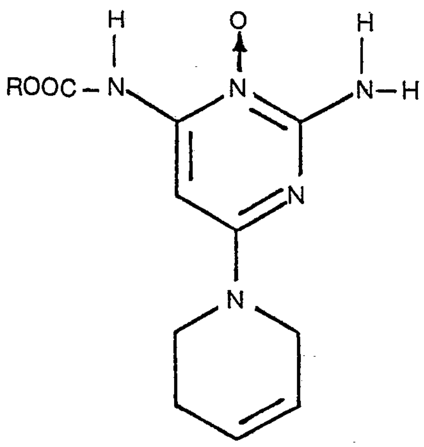

Ia

worin R die obige Bedeutung besitzt.

Wird die Umsetzung hingegen mit einem Ueberschuss an einem Chlorameisensäureester durchgeführt, so erhält man das der Verbindung der Formel Ia entsprechende Dicarbamat der allgemeinen Formel

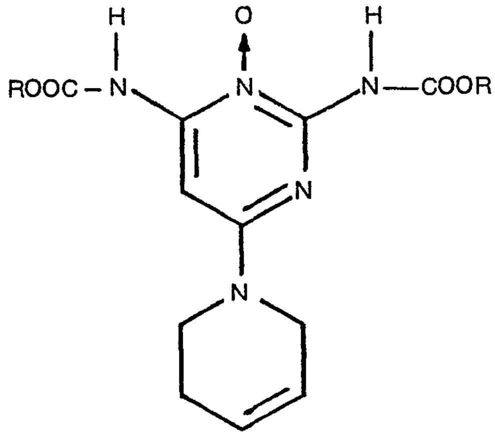

VII

worin R die obige Bedeutung besitzt.

Dieses Dicarbamat kann dann durch Behandlung mit einer wässrigen anorganischen Base, wie Alkalihydroxyde, z.B. Natriumhydroxyd oder Kaliumhydroxyd, Erdalkalihydroxyde, z.B. Bariumhydroxyd oder Calciumhydroxyd, Carbonate, z.B. Kaliumcarbonat oder Natriumcarbonat, oder Bicarbonate, z.B. Natriumcarbonat, und dgl. in die erwünschte Verbindung der Formel Ia übergeführt werden. Die Basenbehandlung erfolgt in Gegenwart eines Lösungsmittels oder Lösungsmittelgemisches bei einer Temperatur zwischen −20° und 50°C, vorzugsweise zwischen 0°C und der Raumtemperatur. Als Lösungsmittel kommen aromatische Kohlenwasserstoffe, wie Benzol, Toluol oder Xylol, chlorierte Kohlenwasserstoffe, wie Methylenchlorid oder Chloroform, Alkohole, wie Methanol oder Aethanol, Aceton, Di-methylformamid, Dimethylsulfoxid und dgl. oder Gemische davon in Frage.

Die Verbindungen der Formel I, worin X Chlor bedeutet, d.h. die Verbindungen der allgemeinen Formel

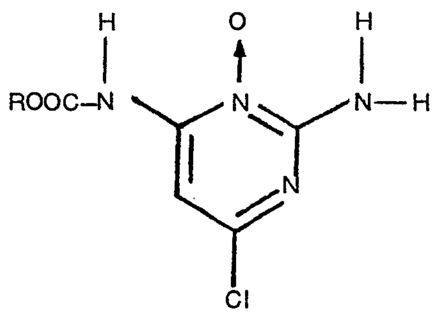

Ib

worin R die obige Bedeutung besitzt, können durch Umsetzen von 2,4-Diamino-6-chlorpyrimidin-3-oxid der Formel

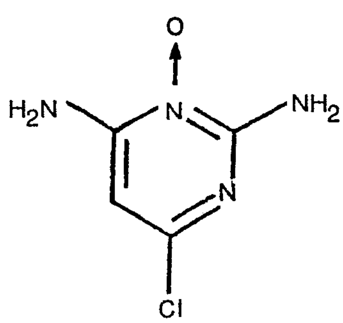

VIII

mit 1 Moläquivalent Chlorameisensäureester der obigen Formel VI hergestellt werden. Die Umsetzung erfolgt unter den für die Umsetzung der Verbindung der Formel III bzw. deren Tautomere der Formeln IV und V mit einem Chlorameisensäureester der Formel VI angegebenen Reaktionsbedingungen.

Gemäss einem Alternativverfahren können die Verbindungen der Formel Ia auch durch Umsetzen eines 2-Amino-4-alkylcarbamat-6-chlorpyrimidin-3-oxids der Formel Ib mit 1,2,5,6-Tetrahydropyridin hergestellt werden. Die Umsetzung erfolgt in einem inerten Lösungsmittel oder Lösungsmittelgemisch. Für den vorliegenden Zweck geeignete Lösungsmittel sind chlorierte Kohlenwasserstoffe wie Methylenchlorid oder Chloroform, aromatische Kohlenwasserstoffe wie Toluol oder Xylol und dgl. oder Gemische davon. Die Umsetzung wird vorzugsweise in einer Inertgasatmosphäre, bevorzugt unter Argon oder

Stickstoff, bei einer Temperatur zwischen 0° und 50°C durchgeführt, vorzugsweise bei der Raumtemperatur. Man kann anstelle eines inerten Lösungsmittels auch überschüssiges 1,2,5,6-Tetrahydropyridin verwenden.

Die Verbindungen der Formel Ia können auch aus den Vergindungen der Formel II hergestellt werden, und zwar durch behandlung mit einer wässrigen anorganischen Base, wie Alkalihydroxyde, z.B. Natriumhydroxyd oder Kaliumhydroxyd, Erdalkalihydroxyde, z.B. Bariumhydroxyd oder Calciumhydroxyd, Carbonate, z.B. Kaliumcarbonat oder Natriumcarbonat, oder Bicarbonate, z.B. Natriumbicarbonat, und dgl. bei einer Temperatur zwischen 0°C und der Raumtemperatur, vorzugsweise bei der Raumtemperatur.

Die Verbindung der Formel III bzw. deren Tautomere der Formeln IV und V sowie die Verbindung der Formel VIII können in Analogie zur Herstellung bekannter Verbindungen hergestellt werden. Herstellungsverfahren sind im nachfolgenden Schema I skizziert. Bezüglich der genauen Reaktionsbedingungen wird auf den Beispielteil verwiesen.

Schema I

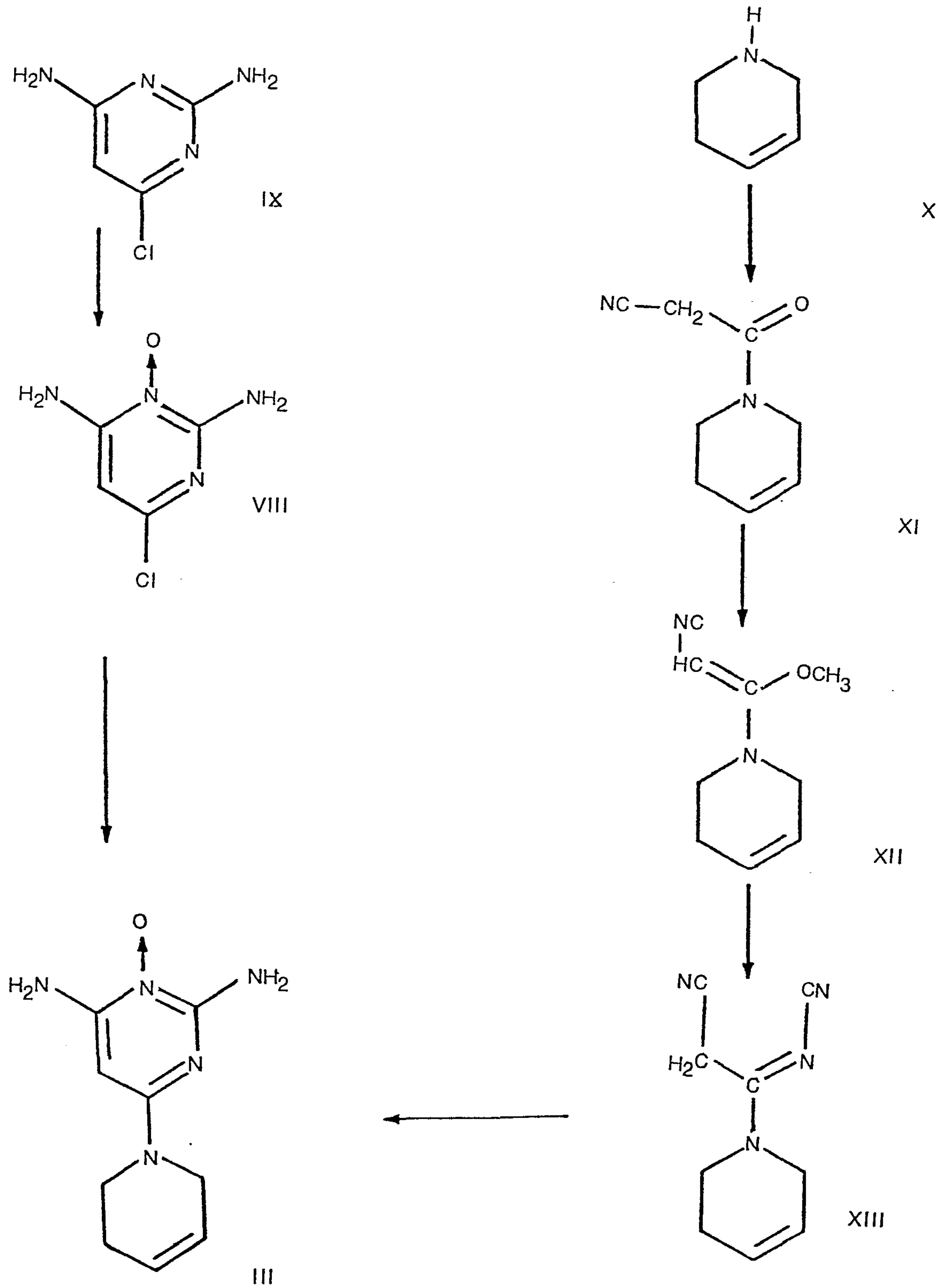

IX

VIII

III

X

XI

XII

XIII

Die Verbindungen der Formel I können zur Herstellung von Verbindungen der Formel II verwendet werden, indem man eine Verbindung der Formel Ia, welche ihrerseits durch Umsetzen einer Verbindung der Formel Ib mit 1,2,5,6-Tetrahydropyridin erhalten werden kann, mit Phosgen umsetzt und erwünschtenfalls eine erhaltene Verbindung in ein Salz oder ein Salz in ein anderes Salz überführt.

Die Umsetzung einer Verbindung der Formel Ia mit Phosgen erfolgt in an sich bekannter Weise in Gegenwart eines Lösungsmittels oder Lösungsmittelgemisches. Als Lösungsmittel kommen

aromatische Kohlenwasserstoffe, wie Benzol, Toluol oder Xylol, chlorierte Kohlenwasserstoffe, wie Methylenchlorid oder Chloroform, und dgl. oder Gemische davon in Frage. Die Reaktion wird zweckmässig bei Atmosphärendruck und einer Temepratur zwischen —20° und 50°C, vorzugsweise bei etwa 0°C in Gegenwart eines säurebindenden Mittels durchgeführt. Geeignete säurebindende Mittel sind tertiäre organische Basen, wie Triäthylamin, Aethyldiisopropylamin oder Pyridin und dgl.

Die Verbindungen der Formel II können in Salze übergeführt werden, beispielsweise durch Behandlung mit einer anorganischen Base oder mit einer organischen Base. Salze der Verbindungen der Formel II können auch durch Umsalzen eines geeigneten Salzes hergestellt werden. Von den Salzen der Verbindungen der Formel II sind die pharmazeutisch verwendbaren bevorzugt.

Die Verbindungen der Formel II sowie deren Salze besitzen langanhaltende wertvolle gefässerweiternde und/oder blutdrucksenkende Eigenschaften und können somit zur Behandlung gefässbedingter Hypertonien oder auch als Vasodilatatoren bei peripheren Durchblutungsstörungen Anwendung finden.

Die blutdrucksenkende Wirkung kann nach folgender Methode an wachen, spontan hypertonen Ratten bestimmt werden:

Der systolische Blutdruck und die Herzfrequenz werden vor Substanzgabe zweimal gemessen. Die Substanzgabe erfolgt mittels Schlundsonde zweimal täglich, morgens und nachmittags. Beide Parameter werden 1, 3, 6 und 24 Stunden nach der Applikation gemessen und die prozentualen Veränderungen zu den Kontrollwerten berechnet.

Der systolische Blutdruck wird indirekt an der Schwanzarterie der Ratte nach der Methode von Gerold et al. gemessen (Helv. Physiol. Act a *24:* 58—69, 1966; Arzneimittelforschung *18;* 1285—1287, 1968).

Die gefässerweiternde Wirkung kann nach folgender Methode an wachen, chronisch implantierten Hunden bestimmt werden:

Weibliche Schäferbastardhunde von ca. 25 kg Körpergewicht werden unter sterilen Kautelen mit einer elektromagnetischen Flowprobe und einem Gefässkonstriktor um die abdominelle Aorta implantiert. Der Nullfluss wird durch Abklemmen des Gefässes mittels des Occluders bestimmt. Die Herzfrequenz, vom pulsatilen Flow getriggert, und der Aortenflow werden kontinuierlich während der ersten drei Stunden nach Substanzgabe und nach 6, 24, 48 und 72 Stunden aufgezeichnet, wobei der Hund jeweils zur Messung ruhig, aber nicht sediert, in einer Versuchsboxe liegt. Die Substanzen werden, in Gelatinekapseln gefüllt, oral verabreicht.

In den nachfolgenden beiden Tabellen sind die erhaltenen Resultate zusammengestellt, wobei jeweils die maximalen prozentualen Abweichungen von den Kontrollwerten sowie die Wirkungsdauer in Stunden, welche als Mittelwerte aus 5 (Ratte) bzw. 3 (Hund) Versuchen berechnet wurden, angegeben sind.

Tabelle I

| Verbindung | Dosis mg/kg p.o. | Blutdruck Δ% | Blutdruck Wirkungsdauer in Stunden | Herzfrequenz Δ% | Herzfrequenz Wirkungsdauer in Stunden |
|---|---|---|---|---|---|
| A | 3 | +9 | >24 | —5 | >24 |
| | 10 | —20 | >24 | +12 | >24 |
| | 30 | —19 | >24 | —7 | >3 |
| | 100 | —29 | >24 | +16 | >24 |

Tabelle II

| Verbindung | Dosis · mg/kg p.o. | AABF* Δ% | AABF* Wirkungsdauer in Stunden | Herzfrequenz Δ% | Herzfrequenz Wirkungsdauer in Stunden |
|---|---|---|---|---|---|
| A | 1 | +21 | >24 | +3 | 6 |
| | 3 | +51 | >24 | +8 | 24 |
| | 10 | +88 | >24 | +19 | >24 |
| | 30 | +111 | >48 | +29 | >72 |

* AABF = abdominal aortic blood flow
A = Methyl 5[3,6-dihydro-1(2H)-pyridyl]-2-oxo-2H-[1,2,4]-oxadiazolo[2,3-a]pyrimidin-7-carbamat

Die Verbindungen der Formel II und deren pharmazeutisch verwendbaren Salze können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial enthalten.

Die tägliche Dosis bei oraler Verabreichung liegt zwischen etwa 10 und 500 mg; bei i.v.-Verabreichung zwischen etwa 1 und 50 mg.

Die nachfolgenden Beispiele 1—6 erläutern die Herstellung von Verbindungen der Formel I und die nachfolgenden Beispiele 7—10 die Verwendung dieser Verbindungen zur Herstellung von Oxadiazolopyrimidinerivaten der Formel II sowie von Salzen davon. Alle Temperaturen sind in Celsiusgraden angegeben. Die Schmelzpunkte sind nicht korrigiert.

Beispiel 1

A) 144,5 g (1 Mol) 2,4-Diamino-6-chlorpyrimidin werden in 2000 ml Aethanol suspendiert. Unter Rühren wird die Suspension auf 35° erwärmt (ca. 15 Minuten), wobei der grösste Teil des Stoffes in Lösung geht. Dieses Gemisch wird dann auf 6—8° abgekühlt, und bei dieser Temperatur werden innerhalb von 40 Minuten 175 ml (ca. 1 Mol) 40% Peressigsäure in Eisessig zugetropft. Nach beendeter Zugabe wird die Mischung noch 30 Minuten bei 6—8° gerührt. Danach lässt man das Gemisch auf Raumtemperatur aufwärmen, und rührt während 3 Stunden bei dieser Temperatur. Darauf gibt man 2000 ml Petroläther hinzu, lässt 1 Stunde weiterrühren und lässt das Gemisch über Nacht stehen. Der ausgefallene Niederschlag wird abfiltriert, mit 200 ml Petroläther nachgewaschen und unter vermindertem Druck getrocknet, wobei man 2,4-Diamino-6-chlorpyrimidin-3-oxid erhält. Umkristallisation liefert analysenreines Produkt vom Schmelzpunkt 193°.

Das 2,4-Diamino-6-chlorpyrimidin-3-oxid kann auch folgendermassen hergestellt werden:

75 g (0,52 Mol) 2,4,-Diamino-6-chlorpyrimidin werden bei 35° in 150 ml Aethanol gelöst. Diese Lösung wird auf −10° abgekühlt und eine Lösung von 100 g (0,57 Mol) 3-Chlor-perbenzoesäure in 500 ml Aethanol innerhalb von 1 Stunde langsam zugetropft. Man rührt die Suspension anschliessend während 7 Stunden bei −10° und lässt sie über Nacht bei 5° stehen. Die Suspension wird mit 24 g Natriumhydroxyd in 100 ml Wasser neutralisiert. Das feste Material wird abfiltriert und aus Aethanol umkristallisiert, wobei man reines 2,4-Diamino-6-chlorpyrimidin-3-oxid erhält.

B) 155 g (0,967 Mol) 2,4-Diamino-6-chlorpyrimidin-3-oxid werden unter Argonatmosphäre mit 640 ml o-Xylol und 260 ml (2,83 Mol) 1,2,5,6-Tetrahydropyridin vermischt und gerührt. Diese Mischung wird dann 30 Minuten zum Rückfluss erhitzt, wobei die Innentemperatur von 115° auf 123° steigt. Die Mischung wird dann auf 5° abgekühlt und mit 40 g Natriumhydroxyd in 400 ml Wasser versetzt und 1 Stunde bei 5° gerührt. Der gebildete Niederschlag wird abfiltriert, mit 200 ml Wasser gewaschen und aus 3000 ml Wasser umkristallisiert, wobei man reines 2,4-Diamino-6-[3,6-dihydro-1(2H)-pyridyl]pyrimidin-3-oxid vom Schmelzpunkt 263—265° (Zers.) erhält.

C) 45 g (0,218 Mol) 2,4-Diamino-6-[3,6-dihydro-1(2H)-pyridyl]pyrimidin-3-oxid werden in 600 ml Methylenchlorid mit 90 ml Triäthylamin vermischt und auf 5° abgekühlt. Unter Rühren werden 90 ml (1,14 Mol) Chlorameisensäuremethylester zugetropft. Die Mischung wird 30 Minuten bei 5° und 18 Stunden bei Raumtemperatur gerührt. Danach wird sie mit 100 ml Methanol versetzt und anschliessend mit 400 ml Methylenchlorid extrahiert, mit Wasser gewaschen, über Natriumsulfat getrocknet und unter vermindertem Druck eingedampft. Umkristallisation des Rückstandes aus Methanol liefert Dimethyl 6-[3,6-dihydro-1(2H)-pyridyl]-2,4-pyrimidin-dicarbamat-3-oxid, Smp. 202—203°. Diese Verbindung kann auch wie folgt hergestellt werden:

20 g (0,0965 Mol) 2,4-Diamino-6-[3,6-dihydro-1(2H)-pyridyl]pyrimidin-3-oxid werden in 100 ml Methylenchlorid und 200 ml Wasser suspendiert und gerührt. Unter Kühlung werden gleichzeitig 25 ml

(0,317 Mol) Chlorameisensäuremethylester in 50 ml Methylenchlorid und 30 ml 28%ige Natronlauge zugetropft, so dass der pH-Wert zwischen 7,5 und 8,5 gehalten wird. Nach beendeter Zugabe wird die Suspension noch 1 Stunde gerührt, und der gebildete Niederschlag anschliessend abfiltriert. Das Filtrat wird mit Methylenchlorid gewaschen und danach mit dem Niederschlag vereinigt. Das ganze wird aus Methylenchlorid/Methanol umkristallisiert, wobei man Dimethyl 6-[3,6-dihydro-1(2H)-pyridyl]-2,4-pyrimidin-dicarbamat-3-oxid erhält, Smp. 202—206° (Zers.).

Das Dimethyl 6-[3,6-dihydro-1(2H)-pyridyl]-2,4-pyrimidin-dicarbamat-3-oxid kann auch noch wie folgt hergestellt werden:

56 g (0,349 Mol) 2,4-Diamino-6-chlorpyrimidin-3-oxid in 500 ml Dimethylformamid und 100 ml Triäthylamin werden auf 0° abgekühlt. Unter Rühren tropft man danach innerhalb einer Stunde 80 ml (1,015 Mol) Chlorameisensäuremethylester zu. Nach beendeter Zugabe wird das Gemisch während 48 Stunden gerührt. Danach wird der Niederschalg abfiltriert und in einem Gemisch von 2500 ml Methylenchlorid und 500 ml Methanol aufgeschlämmt und während 80 Minuten gerührt. Der unlösliche Rückstand wird abfiltriert und getrocknet, wobei man reines Dimethyl 6-chlor-2,4-pyrimidin-dicarbamat-3-oxid, Smp. 204° (Zers.), erhält.

Die organische Phase wird mit Wasser gewaschen und eingeengt, wobei weiteres reines Material gewonnen wird.

Eine Suspension von 10 g (0,036 Mol) Dimethyl 6-chlor-2,4-pyrimidindicarbamat-3-oxid in 40 ml Methylenchlorid wird mit 20 ml (0,22 Mol) 1,2,5,6-Tetrahydropyridin versetzt und unter Argonatmosphäre während 16 Stunden bei Raumtemperatur gerührt. Der gebildete Niederschlag wird abfiltriert und aus einem Gemisch von Methylenchlorid und Methanol umkristallisiert, wobei man reines Dimethyl 6-[3,6-dihydro-1(2H)-pyridyl]-2,4-pyrimidin-dicarbamat-3-oxid erhält, Smp. 203°.

D) 10 g Dimethyl 6-[3,6-dihydro-1(2H)-pyridyl]-2,4-pyrimidindicarbamat-2-oxid werden in 350 ml Chloroform und 100 ml Methanol gelöst. Die erhaltene Lösung wird mit 5 g Natriumcarbonat in 100 ml Wasser versetzt, und das erhaltene Gemisch wird während 80 Stunden bei Raumtemperatur gerührt. Danach wird das Gemisch mit 250 ml Wasser verdünnt und mit konz. Natronlauge auf pH 12,5 gestellt. Das Reaktionsgemisch wird dann während 30 Minuten gerührt, und dann werden die beiden Phasen getrennt. Die wässrige Phase wird noch zweimal mit Methylenchlorid extrahiert. Die kombinierten organischen Extrakte werden über Magnesiumsulfat getrocknet und unter vermindertem Druck zur Trockene eingedampft. Der Rückstand wird unter Verwendung eines Gemisches von Chloroform und Aethanol (90:10) als Eluierungsmittel über Kieselgel chromatographiert, wobei man reines Methyl 2-amino-6-[3,6-dihydro-1(2H)-pyridyl]-4-pyrimidincarbamat-3-oxid vom Smp. 174—176° erhält.

## Beispiel 2

20 g (0,0965 Mol) 2,4-Diamino-6-[3,6-dihydro-1 (2H)-pyridyl]-pyrimidin-3-oxid werden in 140 ml Methylenchlorid und 120 ml Wasser suspendiert und gerührt. Unter Kühlung werden gleichzeitig 8 ml (0,10 Mol) Chlorameisensäuremethylester in 20 ml Methylenchlorid und soviel 28%ige Natronlauge zugetropft, dass der pH-Wert zwischen 7,0 und 7,5 gehalten wird. Nach beendeter Zugabe wird das Gemisch noch 1 Stunde gerührt, und der Rückstand anschliessend abfiltriert. Die beiden erhaltenen Phasen werden getrennt, und die organische Phase wird während 1 Stunde unter Rühren bei 0° mit 150 ml 1N Natronlauge behandelt. Das erhaltene Gemisch wird mit 20 ml Methanol versetzt, und die beiden Phasen getrennt. Die wässrige Phase wird zweimal mit 200 ml Methylenchlorid extrahiert, und die kombinierten organischen Extrakte werden über Natriumsulfat getrocknet. Durch Abdampfen des Lösungsmittels erhält man reines Methyl 2-amino-6-[3,6-dihydro-1 (2H)-pyridyl]-4-pyrimidincarbamat-3-oxid vom Smp. 174—176°.

## Beispiel 3

10 g (0,062 Mol) 2,4-Diamino-6-chlor-pyrimidin-3-oxid werden unter Rühren in 100 ml Dimethylformamid und 10 ml Triäthylamin suspendiert. Die Suspension wird auf ca. 0° abgekühlt, und innerhalb von 10 Minuten werden 5 ml (0,063 Mol) Chlorameisensäuremethylester zugetropft. Das Gemisch wird dann während 45 Minuten bei ca. 0° und anschliessend während 18 Stunden bei Raumtemperatur gerührt. Das ausgefallene Produkt wird abfiltriert, und das Filtrat im Hochvakuum eingeengt. Der Rückstand wird in Methylenchlorid und Methanol gelöst, und das unlösliche Material abfiltriert. Das Filtrat wird nochmals eingeengt, und der Rückstand über Kieselgel chromatographiert, wobei man reines 2-Amino-4-methylcarbamat-6-chlorpyrimidin-3-oxid vom Smp. 220—221° erhält.

## Beispiel 4

1,09 g (0,05 Mol) 2-Amino-4-methylcarbamat-6-chlorpyrimidin-3-oxid werden unter Rühren mit 10 ml Chloroform versetzt. Zu dieser Suspension werden langsam 1,5 ml 1,2,5,6-Tetrahydropyridin in 2 ml Chloroform zugetropft, wobei die Temperatur auf 40° steigt. Das Reaktionsgemisch wird dann während 3 Stunden bei 50° gerührt und danach abgekühlt. Die abgekühlte Lösung wird mit Chloroform verdünnt und mit Wasser gewaschen. Die organische Lösung wird abgetrennt, über Magnesiumsulfat getrocknet und unter vermindertem Druck eingeengt. Der erhaltene Rückstand wird aus Methylenchlorid und Diäthyläther umkristallisiert, wobei man Methyl 2-amino-6-[3,6-dihydro-1 (2H)-pyridyl]-4-pyrimidincarbamat-3-oxid vom Smp. 174—176° erhält.

Beispiel 5

5 g (0,0311 Mol) 2,4-Diamino-6-chlorpyrimidin-3-oxid werden in 50 ml Dimethylformamid und 5 ml Triäthylamin suspendiert und unter Rühren auf 0° abgekühlt. Zu der kalten Suspension tropft man innerhalb von 15 Minuten 4,3 g (0,031 Mol) Chlorameisensäure-2-methoxyäthylester. Das Gemisch wird während 2 Stunden kalt und dann 16 Stunden bei Raumtemperatur gerührt. Das Lösungsmittel wird im Hockvakuum bei 25° abdestilliert, und der Rückstand wird mit Methylenchlorid digeriert. Der erhaltene Niederschlag wird abfiltriert und mit Methylenchlorid, Methanol und Wasser gewaschen. Das Filtrat wird mit Wasser gewaschen und über Natriumsulfat getrocknet. Durch Einengen des Lösungsmittels erhält man reines 2-Methoxyäthyl 2-amino-6-chlor-4-pyrimidincarbamat-3-oxid vom Smp. 176—178°. Kieselgelchromatographische Trennung der Mutterlauge liefert zusätzliches Material.

Beispiel 6

2,5 g (0,094 Mol) 2-Methoxyäthyl 2-amino-6-chlor-4-pyrimidincarbamat-3-oxid werden mit 3 ml 1,2,5,6-Tetrahydropyridin und 25 ml Chloroform unter Argon vermischt. Das Reaktionsgemisch wird während 12 Stunden bei 45° gerührt, dann abgekühlt und mit 15 ml Wasser gewaschen. Das Produkt wird mit Chloroform extrahiert; die organische Phase wird über Natriumsulfat getrocknet und das Lösungsmittel unter vermindertem Druck abdestilliert. Der erhaltene Rückstand wird aus Methylenchlorid/Aether kristallisiert, wobei man reines 2-Methoxyäthyl 2-amino-6-[3,6-dihydro-1(2H)-pyridyl]-4-pyrimidincarbamat von Smp. 1.36° erhält.

Beispiel 7

53 mg (0,2 mMol) Methyl 2-amino-6-[3,6-dihydro-1 (2H)-pyridyl]-4-pyrimidincarbamat-3-oxid werden in 5 ml Methylenchlorid gelöst. Diese Lösung wird auf 0° abgekühlt und mit 0,2 ml Triäthylamin und 0,12 ml (0,22 mMol) 20% Phosgen in Toluol versetzt. Das Gemisch wird dann während 30 Minuten gerührt, danach mit 0,5 ml konz. Natronlauge und 10 ml Wasser versetzt. Nach 15-minütigem Rühren werden die beiden Phasen getrennt, und die organische Phase nochmals mit Wasser gewaschen. Die kombinierten wässrigen Phasen werden mit Salzsäure angesäuert, wobei man reines Methyl 5-[3,6-dihydro-1 (2H)-pyridyl]-2-oxo-2H-[1,2,4]-oxadiazolo[2,3-a]pyrimidin-7-carbamat vom Smp. 213—214° erhält.

Beispiel 8

2 g (6,9 mMol) Methyl 5-[3,6-dihydro-1 (2H)-pyridyl]-2-oxo-2H-[1,2,4]oxadiazolo[2,3-a]pyrimidin-7-carbamat werden in Acetonitril aufgeschlämmt und mit 1,6 g 3-Azabicyclo[3.2.2]-nonan in 20 ml Acetonitril versetzt. Es entsteht zunächst eine klare Lösung, wobei jedoch sehr rasch das entsprechende 3-Azabicyclo[3.2.2]nonansalz ausfällt, dessen Struktur durch Röntgenstrukturanalyse bestätigt wurde. Durch Umkristallisation erhält man das reine Salz vom Schmelzpunkt 164—168°.

Beispiel 9

3 g (10,5 mMol) methyl 5-[3,6-dihydro-1 (2H)-pyridyl]-2-oxo-2H-[1,2,4]oxadiazolo[2,3-a]pyrimidin-7-carbamat werden in 2N Natronlauge gelöst und stehen gelassen. Beim Abkühlen fällt das entsprechende Natriumsalz aus, das aus Acetronitril und Wasser umkristallisiert wird und welches sich ab 145° zu zersetzen beginnt.

Beispiel 10

2 g (0,0065 Mol) 2-Methoxyäthyl 2-amino-6-[2,6-dihydro-1 (2H)-pyridyl]-4-pyrimidincarbamat-3-oxid werden bei 0° in 15 ml Chloroform und 3,7 ml Triäthylamin gelöst. Das kalt gerührte Gemisch wird mit 3,2 ml (0,0061 Mol) 20% Phosgen in Toluol versetzt. Das Gemisch wird dann während 4 Stunden gerührt, mit Salzsäure angesäuert, mit Wasser gewaschen und mit Methylenchlorid extrahiert. Die organische Phase wird über Magnesiumsulfat getrocknet, und das Lösungsmittel wird unter vermindertem druck abdestilliert. Der erhaltene Rückstand wird aus Methylenchlorid und Aether kristallisiert, wobei man reines 2-Methoxyäthyl 5-[3,6-dihydro-1 (2H)-pyridyl]-2-oxo-2H-[1,2,4]oxadiazolo[2,3-a]pyrimidin-7-carbamat vom Smp. 195—196° erhält.

**Patentansprüche fur die benannten Vertragsstaaten: BE CH DE FR GB IT LU NL SE**

1. Verbindungen der allgemeinen Formel

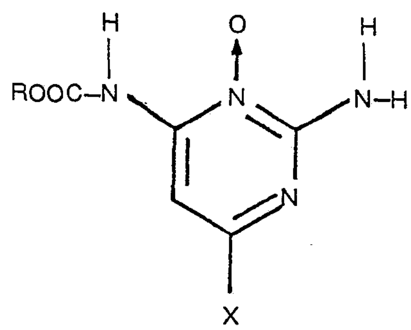

I

worin R $C_1$—$C_8$-Alkyl oder $C_1$—$C_8$-Alkoxy-$C_1$—$C_8$-alkyl und X 1,2,5,6-Tetrahydropyridin-1-yl oder Chlor bedeuten.

2. Verbindungen gemäss Anspruch 1, worin R $C_1$—$C_8$-Alkyl bedeutet,

3. Verbindungen gemäss Anspruch 2, worin R. Alkyl mit 1—4 Kohlenstoffatomen bedeutet.

4. Verbindungen gemäss Anspruch 3, worin R Methyl bedeutet.

5. Verwendung von Verbindungen gemäss einem der Ansprüche 1—4 zur Herstellung von Verbindungen der allgemeinen Formel

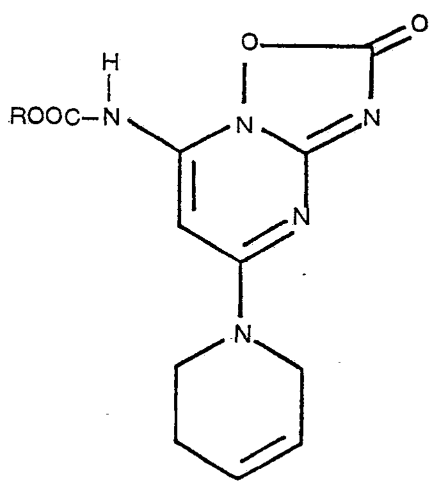

II

worin R die in Anspruch 1 angegebene Bedeutung besitzt, sowie von Salzen davon.

**Patentansprüche für den Vertragsstaat: AT**

1. Verfahren zur Herstellung von neuen Oxadiazolopyrimidinderivaten der allgemeinen Formel

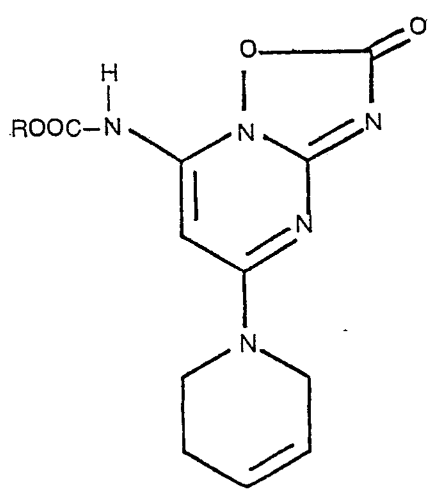

II

worin R $C_1$—$C_8$-Alkyl oder $C_1$—$C_8$-Alkoxy-$C_1$—$C_8$-alkyl bedeutet, sowie von Salzen davon, dadurch gekennzeichnet, dass man eine Verbindung der allgemeinen Formel

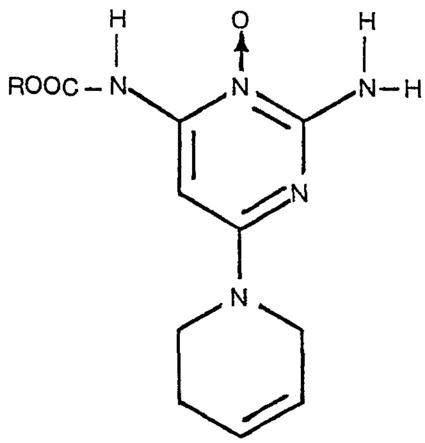

Ia

worin R die obige Bedeutung besitzt, mit Phosgen umsetzt und erwünschtenfalls eine erhaltene Verbindung der Formel I in ein Salz oder ein Salz in ein anderes Salz überführt.

2. Verfahren nach Anspruch 1, dadurch gekennzeichnet, dass R $C_1$—$C_8$-Alkyl bedeutet.

3. Verfahren nach Anspruch 2, dadurch gekennzeichnet, dass der Alkylrest 1—4 Kohlenstoffatome aufweist.

4. Verfahren nach Anspruch 3, dadurch gekennzeichnet, dass man Methyl 5-[3,6-dihydro-1 (2H)-pyridyl]-2-oxo-2H-[1,2,4]-oxadiazolo[2,3-a]pyrimidin-7-carbamat herstellt.

**Claims for the Contracting States: BE CH DE FR GB IT LU NL SE**

1. Compounds of the general formula

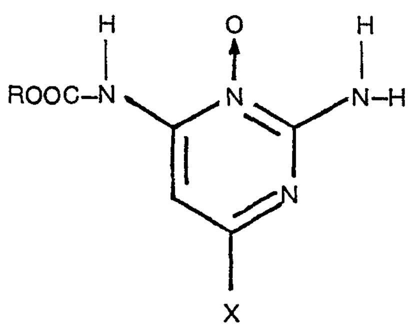

I

wherein R signifies $C_1$—$C_8$-alkyl or $C_1$—$C_8$-alkoxy-$C_1$—$C_8$-alkyl and X signifies 1,2,5,6-tetrahydropyridin-1-yl or chlorine.

2. Compounds in accordance with claim 1, wherein R signifies $C_1$—$C_8$-alkyl.

3. Compounds in accordance with claim 2, wherein R signifies alkyl with 1—4 carbon atoms.

4. Compounds in accordance with claim 3, wherein R signifies methyl.

5. The use of compounds in accordance with one of claims 1—4 for the manufacture of compounds of the general formula

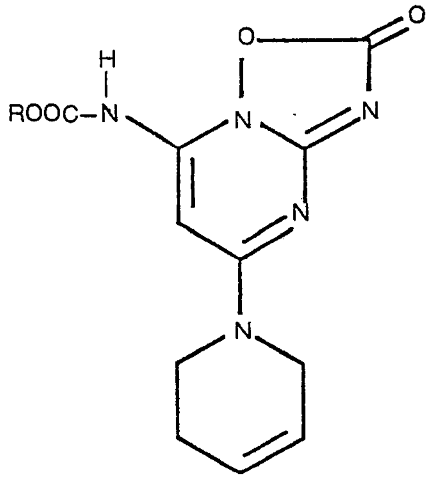

II

wherein R has the significance given in claim 1, as well as of salts thereof.

**Claims for the Contracting State: AT**

1. Process for the manufacture of novel oxadiazolopyrimidine derivatives of the general formula

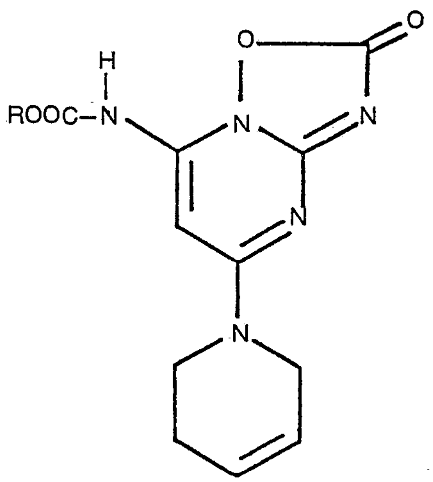

II

wherein R signifies $C_1$—$C_8$-alkyl or $C_1$—$C_8$-alkoxy-$C_1$—$C_8$-alkyl, as well as of salts thereof, characterized by reacting a compound of the general formula

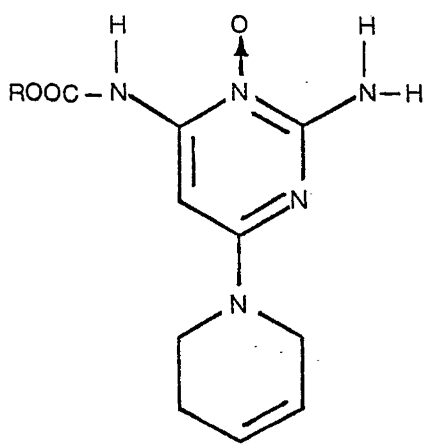

Ia

wherein R has the above significance, with phosgene and, if desired, converting a compound of formula I obtained into a salt or converting a salt into another salt.

2. Process according to claim 1, characterized in that R signifies $C_1$—$C_8$-alkyl.

3. Process according to claim 2, characterized in that the alkyl residue has 1—4 carbon atoms.

4. Process according to claim 3, characterized in that methyl 5-[3,6-dihydro-1 (2H)-pyridyl]-2-oxo-2H-[1,2,4]oxadiazolo[2,3-a]pyrimidine-7-carbamate is manufactured.

**Revendications pour les Etats contractants: BE, CH, DE, FR, GB, IT, LU, NL, SE**

1. Composés de formule générale

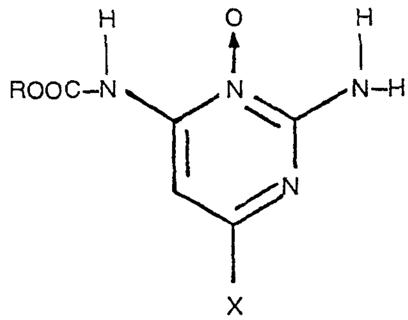

I

dans laquelle R représente un groupe alkyle en $C_1$—$C_8$ ou (alcoxy en $C_1$—$C_8$)-alkyle en $C_1$—$C_8$ et X représente un groupe 1,2,5,6-tétrahydropyridine-1-yle ou le chlore.

2. Composés selon la revendication 1, dans lesquels R est un groupe alkyle en $C_1$—$C_8$.

3. Composés selon la revendication 2, dans lesquels R est un groupe alkyle en $C_1$—$C_4$.

4. Composés selon la revendication 3, dans lesquels R représente un groupe méthyle.

5. Utilisation des composés selon l'une des revendications 1 à 4 pour la préparation de composés de formule générale

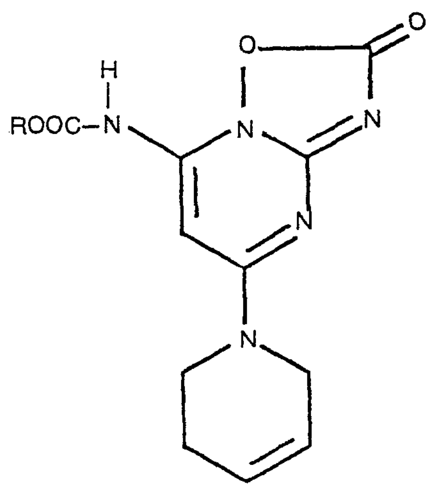

II

dans laquelle R a la signification indiquée dans la revendication 1, et de leurs sels.

**Revendications pour l'Etat contractant: AT**

1. Procédé de préparation de nouveaux dérivés de l'oxadiazolopyrimidine, répondant à la formule générale

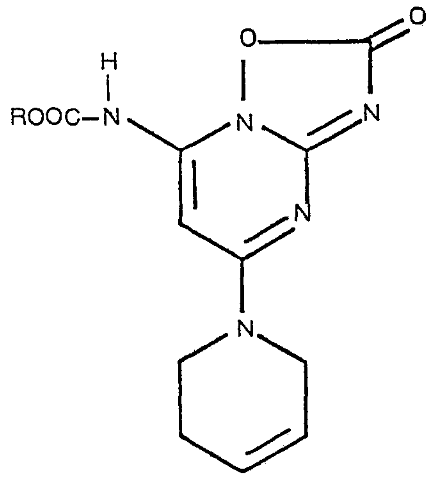

II

dans laquelle R représente un groupe alkyle en C1—C8 ou (alcoxy en C1—C8)-alkyle en C1—C8, et de leurs sels, caractérisé en ce que l'on fait réagir un composé de formule générale

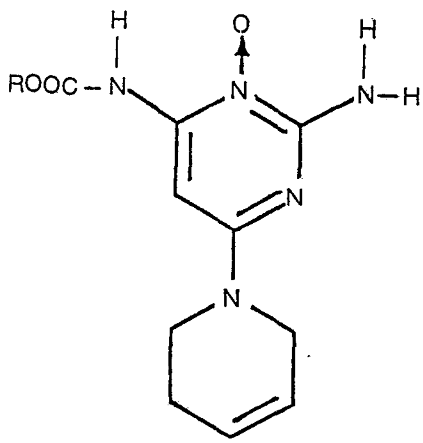

Ia

dans laquelle R a la signification indiquée ci-dessus, avec le phosgène et si on le désire on convertit un composé obtenu de formule I en un sel ou un sel en un autre sel.

2. Procédé selon la revendication 1, caractérisé en ce que R représente un groupe alkyle en C1—C8.

3. Procédé selon la revendication 2, caractérisé en ce que le groupe alkyle contient de 1 à 4 atomes de carbone.

4. Procédé selon la revendication 3, caractérisé en ce que l'on prépare le 5-[3,6-dihydro-1 (2H)-pyridyl]-2-oxo-2H-[1,2,4]-oxadiazolo[2,3-a]pyrimidine-7-carbamate de méthyle.